# EUROPEAN PATENT APPLICATION

(11) **EP 1 757 593 A1**
(43) Date of publication of application: **28.02.2007**
(21) Application number: 05737159.3
(22) Date of filing: 27.04.2005
(51) Int. Cl.: C07D 239/42, A61K 31/506, A61P 1/16

(54) **THERAPEUTIC AGENT FOR NONVIRAL HEPATITIS**

(30) Priority: 27.04.2004 JP 2004131116
(71) Applicant: Mitsubishi Pharma Corporation, Osaka-shi, Osaka 541-0046 (JP)
(72) Inventor: FUKUDA, Tetsuko, MITSUBISHI PHARMA CORPORATION, Chuo-ku, Tokyo 1038405 (JP); MOGAMI, Akira, MITSUBISHI PHARMA CORPORATION, Chuo-ku, Tokyo 1038405 (JP); HISADOME, Masao, MITSUBISHI PHARMA CORPORATION, Chuo-ku, Tokyo 1038405 (JP); ADACHI, Kunitomo, MITSUBISHI PHARMA CORPORATION, Chuo-ku, Tokyo 1038405 (JP); KOMATSU, Hirotsu, Yokohama-shi, Kanagawa 225-0015 (JP)
(74) Representative: von Kreisler, Alek
(86) International application number: PCT/JP2005/007951
(87) International publication number: WO 2005/103013

(57) **Abstract**

The present invention provides a therapeutic drug for non-viral hepatitis, which contains a piperazine compound represented by the following formula (I) 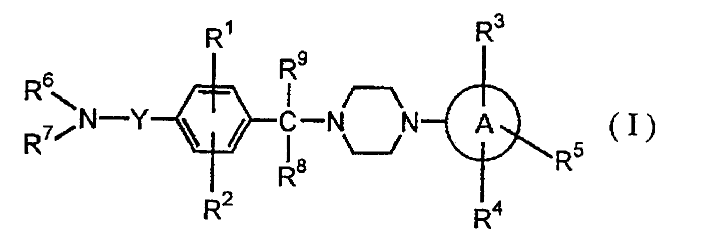 or a pharmaceutically acceptable salt thereof as an active ingredient.

## Description

### Technical Field

The present invention relates to a novel therapeutic drug for non-viral hepatitis.

### Background Art

As protein involved in the expression of biological functions of living organisms such as immune response, inflammatory reaction and the like, many cytokines have been found. Of the cytokines, tumor necrosis factor alpha (hereinafter to be referred to as TNF-α) plays a key role in various autoimmune diseases. It has been reported in recent years that pentoxifylline that inhibits TNF-α (non-patent reference 1) and anti-TNF-α antibody (non-patent reference 2) are effective for severe alcoholic hepatitis in non-viral hepatitis. While such effective pharmaceutical agents have been discovered, a specific drug for severe alcoholic hepatitis has not been found.

In animal experiments, pentoxifylline and anti-TNF-α antibody have been reported to inhibit the progress of concanavalin A-induced hepatitis in mouse (non-patent reference 3 and non-patent reference 4). The administration condition of these drugs is prophylactic administration of the drug before injection of concanavalin A stimulating agent, and there is no report on the effectiveness of the drug by therapeutic administration after injection of a stimulating agent. In addition, of the conventional non-viral hepatitis therapeutic agents other than these pharmaceutical agents, no pharmaceutical agent showed a clear effect by therapeutic administration to concanavalin A hepatitis model, leaving room for improvement in the pharmacological action. In consideration of the clinical situations where a drug therapy is applied to patients who developed non-viral hepatitis such as alcoholic hepatitis and the like, the development of a pharmaceutical agent having a treatment effect on concanavalin A hepatitis in animal experiments has been demanded.

As a compound inhibiting TNF-α, piperazine compounds as those shown in patent reference 1 are currently known. Patent reference 1 describes that the compounds are effective for various diseases associated with abnormal TNF-α production or TNF-α-mediated diseases, and effective for viral hepatitis. However, it has not been described at all that the compound is effective for non-viral hepatitis, or that it has a treatment effect on non-viral hepatitis.
patent reference 1: WO99/19301
non-patent reference 1: Akriviadis, G.E., et al., Gastroenterology, 119, 1637-1648 (2000)
non-patent reference 2: Tilg, H., et al., Journal of Hepatology, 38, 419-425 (2003)
non-patent reference 3: Shirin, H., et al., Journal of Hepatology, 29, 60-67 (1998)
non-patent reference 4: Gantner, F., et al., Hepatology, 21, 190-198 (1995)

### Disclosure of the Invention

### Problems to be Solved by the Invention

The present inventors have conducted intensive studies and confirmed that pentoxifylline and anti-TNF-α, antibody, which have been known to have a prophylactic effect on non-viral hepatitis, do not inhibit progress of concanavalin A-induced hepatitis in mouse by a therapeutic administration thereof. On the other hand, they have confirmed that a certain kind of piperazine compound inhibits progress of concanavalin A-induced hepatitis in mouse by a therapeutic administration, unlike pentoxifylline and anti-TNF-α, antibody. As a result, they have found that a novel therapeutic drug for non-viral hepatitis can be provided, which resulted in the completion of the present invention.

### Means of Solving the Problems

Accordingly, the present invention provides the following.
(1) A therapeutic drug for non-viral hepatitis comprising, as an active ingredient, a piperazine compound represented by the following formula (I)

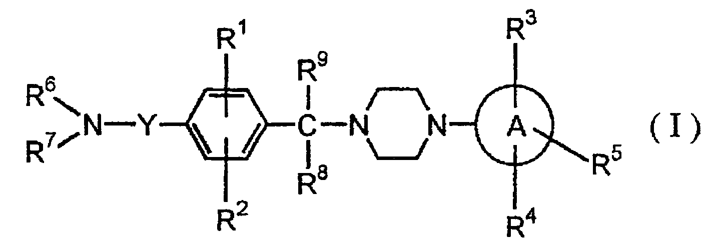

wherein R¹ and R² are the same or different and each is an amino, a nitro, a hydroxy or a cyano, which is mono- or di-substituted by a group selected from a hydrogen, a halogen, a lower alkyl, a lower alkoxy, an amino, a lower alkyl and a lower acyl;

R³, R⁴ and R⁵ are the same or different and each is an amino or a hydroxy, which is mono- or di-substituted by a group selected from a hydrogen, a halogen, a lower alkyl, a lower alkoxy, a nitro, an amino, a lower alkyl and a lower acyl;

R⁶ and R⁷ are the same or different and each is a hydrogen, a lower alkyl, a lower alkyl substituted by 1 to 3 halogens, an aralkyl, an acyl or a lower acyl substituted by 1 to 3 halogens;

R⁸ and R⁹ are the same or different and each is a hydrogen or a lower alkyl; and

Y is a group represented by

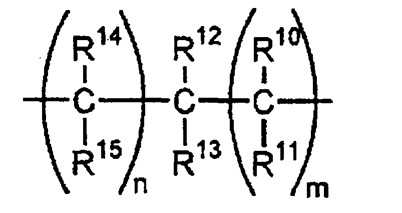

wherein R¹⁰ and R¹¹ are the same or different and each is a hydrogen or a lower alkyl;

R¹² and R¹³ are the same or different and each is a hydrogen or a lower alkyl, or R¹² and R¹³ in combination form alkylene;

R¹⁴ and R¹⁵ are the same or different and each is a hydrogen or a lower alkyl;

m is an integer of 0-2, n is an integer of 0-2, and 0≤m+n≤2; and

ring A is a phenyl, a pyrimidyl, a thiazolyl, a pyridyl, a pyradyl or an imidazolyl,
or a pharmaceutically acceptable salt thereof.
(2) The therapeutic drug of the above-mentioned (1), wherein the active ingredient is a compound of the formula (I) wherein R¹ and R² are each a hydrogen, R³, R⁴ and R⁵ are the same or different and each is a halogen or a lower alkoxy, R⁶ and R⁷ are each an acyl, R⁸ and R⁹ are the same or different and each is a hydrogen or a lower alkyl, R¹⁰ and R¹¹ are each a hydrogen, R¹⁴ and R¹⁵ are each a hydrogen, ring A is a phenyl, a pyrimidyl, a thiazolyl or a pyridyl, or a pharmaceutically acceptable salt thereof.

(3) The therapeutic drug of the above-mentioned (1), wherein the active ingredient is the following compound:
   N-(4-((4-phenylpiperazin-1-yl)methyl)phenylmethyl)acetamide;
   N-(4-((4-(4-fluorophenyl)piperazin-1-yl)methyl)phenylmethyl)acetamide;
   N-(4-((4-(2-fluorophenyl)piperazin-1-yl)methyl)phenylmethyl)acetamide;
   N-(3-(4-((4-phenylpiperazin-1-yl)methyl)phenyl)propyl)acetamide;
   N-(4-((4-(2,4-difluorophenyl)piperazin-1-yl)methyl)phenylmethyl)acetamide;
   N-(4-(1-(4-(4-fluorophenyl)piperazin-1-yl)ethyl)phenylmethyl)acetamide;
   N-(4-((4-(pyridin-2-yl)piperazin-1-yl)methyl)phenylmethyl)acetamide;
   N-(4-((4-(pyrimidin-2-yl)piperazin-1-yl)methyl)phenylmethyl)acetamide;
   N-(4-((4-(pyridin-3-yl)piperazin-1-yl)methyl)phenylmethyl)acetamide;
   N-(4-((4-(thiazol-2-yl)piperazin-1-yl) methyl) phenylmethyl) acetamide;
   N-(1-methyl-1-(4-((4-(4-fluorophenyl)piperazin-1-yl) methyl) phenyl) ethyl) acetamide;
   N-(1-(4-((4-(2,-4-difluorophenyl)piperaz-in-1-yl)methyl)phenyl)ethyl)acetamide;
   N-(1-(4-((4-(2,4-difluorophenyl)piperazin-1-yl)methyl)phenyl)propyl)acetamide;
   N-(1-(4-((4-(4-fluorophenyl)piperazin-1-yl)methyl)phenyl)ethyl)acetamide;
   N-(1-(4-((4-phenylpiperazin-1-yl)methyl)phenyl)cyclopropyl)acetamide;
   N-(1-(4-((4-(4-fluorophenyl)piperazin-1-yl)methyl)phenyl)cyclopropyl)acetamide;
   N-(1-(4-((4-(2,4-difluorophenyl)piperazin-1-yl)methyl)phenyl)cyclopropyl)acetamide;
   N-(4-((4-(6-fluoropyridin-2-yl)piperazin-1-yl)methyl)phenylmethyl)acetamide;
   N-(4-((4-(pyrimidin-2-yl)piperazin-1-yl)methyl)phenylmethyl)acetamide;
   N-(1-(4-(1-(4-phenylpiperazin-1-yl)ethyl)phenyl)-1-methylethyl)acetamide;
   N-(1-(4-(1-(4-(pyrimidin-2-yl)piperazin-1-yl)ethyl)phenyl)cyclopropyl)acetamide;
   N-(1-(4-((4-(pyrimidin-2-yl)piperazin-1-yl)methyl)phenyl)cyclopropyl)acetamide;
   N-(1-(4-((4-(pyrimidin-2-yl)piperazin-1-yl)methyl)phenyl)ethyl)acetamide;
   N-(4-((4-(4,6-dimethoxypyrimidin-2-yl)piperazin-1-yl) methyl) phenylmethyl) acetamide;
   N-(1-methyl-1-(4-((4-(pyrimidin-2-yl)piperazin-1-yl)methyl)phenyl)ethyl)acetamide;
   N-(1-methyl-1-(4-((4-(thiazol-2-yl)piperazin-1-yl)methyl)phenyl)ethyl)acetamide;
   N-(1-(4-((4-(thiazol-2-yl)-piperazin-1-yl)methyl)phenyl)cyclopropyl)acetamide;
   N-(1-(4-((4-(thiazol-2-yl)piperazin-1-yl)methyl)phenyl)ethyl)acetamide;
   N-(1-(4-((4-(6-fluoropyridin-2-yl)piperazin-1-yl)methyl)phenyl)-1-methylethyl)-acetamide;
   N-(1-(4-((4-(6-fluoropyridin-2-yl)piperazin-1-yl)methyl)phenyl)ethyl)acetamide;
   N-(1-(4-((4-(pyridin-2-yl)piperazin-1-yl)methyl)phenyl)ethyl)acetamide;
   N-(1-methyl-1-(4-((4-(pyridin-2-yl)piperazin-1-yl)methyl)phenyl)ethyl)acetamide;
   N-(1-(4-(1-(4-(pyridin-2-yl)piperazin-1-yl)ethyl)phenyl)-1-methylethyl)acetamide;
   N-(1-(4-((4-(pyridin-2-yl)piperazin-1-yl)methyl)phenyl)cyclopropyl)acetamide;
   N-(1-(4-((4-(6-fluoropyridin-2-yl)piperazin-1-yl)methyl)phenyl)cyclopropyl)acetamide;
   (S)-N-(1-(4-((4-(4-fluorophenyl)piperazin-1-yl)methyl)phenyl)ethyl)acetamide;
   (R)-N-(1-(4-((4-(4-fluorophenyl)piperazin-1-yl)methyl)phenyl)ethyl)acetamide;
   N-(4-(1-(4-(pyridin-2-yl)piperazin-1-yl)ethyl)phenylmethyl)acetamide;
   N-(1-(4-(1-(4-(6-fluoropyridin-2-yl)piperazin-1-yl)ethyl)phenyl)-1-methylethyl)acetamide; or
   N-(1-(4-(1-(4-(6-fluoropyridin-2-yl)piperazin-1-yl)ethyl)phenyl)methyl)acetamide,
   or a pharmaceutically acceptable salt thereof.
(4) The therapeutic drug of the above-mentioned (1), wherein the active ingredient is N-(1-(4-((4-(pyrimidin-2-yl)piperazin-1-yl)methyl)phenyl)cyclopropyl)acetamide, or a pharmaceutically acceptable salt thereof.
(5) The therapeutic drug of the above-mentioned (1), wherein the active ingredient is N-(1-(4-((4-(pyrimidin-2-yl)piperazin-1-yl)methyl)phenyl)cyclopropyl)acetamide hydrochloride.
(6) The therapeutic drug of any of the above-mentioned (1) to (5), wherein the non-viral hepatitis is selected from acute hepatitis, hepatic encephalopathy, cirrhosis, primary biliary hepatitis, autoimmune hepatitis, alcoholic hepatitis and nonalchoholic steatohepatitis.
(7) A hepatic function-improving drug or hepatoprotector for liver transplant patient, which comprises, as an active ingredient, a piperazine compound represented by the following formula (I)

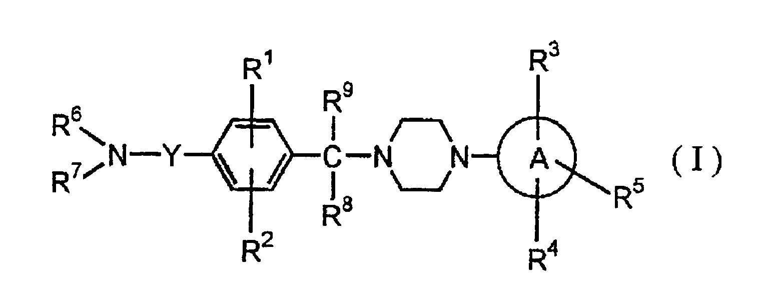

wherein R¹ and R² are the same or different and each is an amino, a nitro, a hydroxy or a cyano, which is mono- or di-substituted by a group selected from a hydrogen, a halogen, a lower alkyl, a lower alkoxy, an amino, a lower alkyl and a lower acyl;

R³, R⁴ and R⁵ are the same or different and each is an amino or hydroxy, which is mono- or di-substituted by a group selected from a hydrogen, a halogen, a lower alkyl, a lower alkoxy, a nitro, an amino, a lower alkyl and lower an acyl;

R⁶ and R⁷ are the same or different and each is a hydrogen, a lower alkyl, a lower alkyl substituted by 1 to 3 halogens, an aralkyl, an acyl or a lower acyl substituted by 1 to 3 halogens;

R⁸ and R⁹ are the same or different and each is a hydrogen or a lower alkyl;

Y is

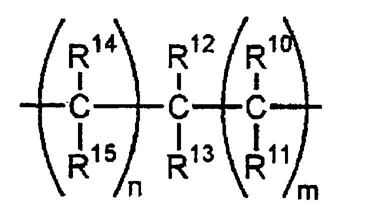

wherein R¹⁰ and R¹¹ are the same or different and each is a hydrogen or a lower alkyl;

R¹² and R¹³ are the same or different and each is a hydrogen or a lower alkyl, or R¹² and R¹³ in combination form alkylene;

R¹⁴ and R¹⁵ are the same or different and each is a hydrogen or a lower alkyl;

m is an integer of 0-2, n is an integer of 0-2, and 0≤m+n≤2; and

ring A is a phenyl, a pyrimidyl, a thiazolyl, a pyridyl, a pyradyl or an imidazolyl,
or a pharmaceutically acceptable salt thereof.
(8) The hepatic function-improving drug or hepatoprotector of the above-mentioned (7), wherein the active ingredient is a compound of the formula (I), wherein R¹ and R² are each a hydrogen, R³, R⁴ and R⁵ are the same or different and each is a halogen or a lower alkoxy, R⁶ and R⁷ are each an acyl, R⁸ and R⁹ are the same or different and each is a hydrogen or a lower alkyl, R¹⁰ and R¹¹ are each a hydrogen, R¹⁴ and R¹⁵ are each a hydrogen, ring A is a phenyl, a pyrimidyl, a thiazolyl or a pyridyl, or a pharmaceutically acceptable salt thereof.
(9) The hepatic function-improving drug or hepatoprotector of the above-mentioned (7), wherein the active ingredient is the following compound:
   N-(4-((4-phenylpiperazin-1-yl)-mathyl)phenylmethyl)acetamide;
   N-(4-((4-(4-fluorophenyl)piperazin-1-yl)methyl)phenylmethyl)acetamide;
   N-(4-((4-(2-fluorophenyl)piperazin-1-yl)methyl)phenylmethyl)acetamide;
   N-(3-(4-((4-phenylpiperazin-1-yl) methyl) phenyl) propyl) acetamide;
   N-(4-((4-(2,4-difluorophenyl)piperazin-1-yl)methyl)phenylmethyl)acetamide;
   N-(4-(1-(4-(4-fluorophenyl)piperazin-1-yl)ethyl)phenylmethyl)acetamide;
   N-(4-((4-(pyridin-2-yl)piperazin-1-yl)methyl)phenylmethyl)acetamide;
   N-(4-((4-(pyrimidin-2-yl)piperazin-1-yl)methyl)phenylmethyl)acetamide;
   N-(4-((4-(pyridin-3-yl)piperazin-1-yl)methyl)phenylmethyl)acetamide;
   N-(4-((4-(thiazol-2-yl)piperazin-1-yl)methyl)phenylmethyl)acetamide;
   N-(1-methyl-1-(4-((4-(4-fluorophenyl)piperazin-1-yl)methyl)phenyl)ethyl)acetamide;
   N-(1-(4-((4-(2,4-difluorophenyl)piperazin-1-yl)methyl)phenyl)ethyl)acetamide;
   N-(1-(4-((4-(2,4-difluorophenyl)piperazin-1-yl)methyl)phenyl)propyl)acetamide;
   N-(1-(4-((4-(4-fluorophenyl)piperazin-1-yl)methyl)phenyl)ethyl)acetamide;
   N-(1-(4-((4-phenylpiperazin-1-yl)methyl)phenyl)cyclopropyl)acetamide;
   N-(1-(4-((4-(4-fluorophenyl)piperazin-1-yl)methyl)phenyl)cyclopropyl)acetamide;
   N-(1-(4-((4-(2,4-difluorophenyl)piperazin-1-yl)methyl)phenyl)cyclopropyl)acetamide;
   N-(4-((4-(6-fluoropyridin-2-yl)piperazin-1-yl)methyl)phenylmethyl)acetamide;
   N-(4-((4-(pyrimidin-2-yl)piperazin-1-yl)methyl)phenylmethyl)acetamide;
   N-(1-(4-(1-(4-phenylpiperazin-1-yl)ethyl)phenyl)-1-methylethyl)acetamide;
   N-(1-(4-(1-(4-(pyrimidin-2-yl)piperazin-1-yl)ethyl)phenyl)cyclopropyl)acetamide;
   N-(1-(4-((4-(pyrimidin-2-yl)piperazin-1-yl)methyl)phenyl)cyclopropyl)acetamide;
   N-(1-(4-((4-(pyrimidin-2-yl)piperazin-1-yl)methyl)phenyl)ethyl)acetamide;
   N-(4-((4-(4,6-dimethoxypyrimidin-2-yl)piperazin-1-yl)methyl)phenylmethyl)acetamide;
   N-(1-methyl-1-(4-((4-(pyrimidin-2-yl)piperazin-1-yl)methyl)phenyl)ethyl)acetamide;
   N-(1-methyl-1-(4-((4-(thiazol-2-yl)piperazin-1-yl)methyl)phenyl)ethyl)acetamide;
   N-(1-(4-((4-(thiazol-2-yl)piperazin-1-yl)methyl)phenyl)cyclopropyl)acetamide;
   N-(1-(4-((4-(thiazol-2-yl)piperazin-1-yl)methyl)phenyl)ethyl)acetamide;
   N-(1-(4-((4-(6-fluoropyridin-2-yl)piperazin-1-yl)methyl)phenyl)-1-methylethyl)acetamide;
   N-(1-(4-((4-(6-fluoropyridin-2-yl)piperazin-1-yl)methyl)phenyl)ethyl)acetamide;
   N-(1-(4-((4-(pyridin-2-yl)piperazin-1-yl)methyl)phenyl)ethyl)acetamide;
   N-(1-methyl-1-(4-((4-(pyridin-2-yl)piperazin-1-yl)methyl)phenyl)ethyl)acetamide;
   N-(1-(4-(1-(4-(pyridin-2-yl)piperazin-1-yl)ethyl)phenyl)-1-methylethyl)acetamide;
   N-(1-(4-((4-(pyridin-2-yl)piperazin-1-yl)methyl)phenyl)cyclopropyl)acetamide;
   N-(1-(4-((4-(6-fluoropyridin-2-yl)piperazin-1-yl)methyl)phenyl)cyclopropyl)acetamide;
   (S)-N-(1-(4-((4-(4-fluorophenyl)piperazin-1-yl)methyl)phenyl)ethyl)acetamide;
   (R)-N-(1-(4-((4-(4-fluorophenyl)piperazin-1-yl)methyl)phenyl)ethyl)acetamide;
   N-(4-(1-(4-(pyridin-2-yl)piperazin-1-yl)ethyl)phenylmethyl)acetamide;
   N-(1-(4-(1-(4-(6-fluoropyridin-2-yl)piperazin-1-yl)ethyl)phenyl)-1-methylethyl)acetamide; or
   N-(1-(4-(1-(4-(6-fluoropyridin-2-yl)piperazin-1-yl)ethyl)phenyl)methyl)acetamide,
   or a pharmaceutically acceptable salt thereof.
(10) The hepatic function-improving drug or hepatoprotector of the above-mentioned (7), wherein the active ingredient is N-(1-(4-((4-(pyrimidin-2-yl)piperazin-1-yl)methyl)phenyl)cyclopropyl)acetamide or a pharmaceutically acceptable salt thereof.
(11) The hepatic function-improving drug or hepatoprotector of the above-mentioned (7), wherein the active ingredient is N-(1-(4-((4-(pyrimidin-2-yl)piperazin-1-yl)methyl)phenyl)cyclopropyl)acetamide hydrochloride.

### Brief Description of the Drawings

Fig. 1 shows the effect of pentoxifylline (Fig. 1-A) and the effect of anti-TNF-α antibody (Fig. 1-B), in hepatitis model of Example 1.
Fig. 2 shows the effect of the test compound in hepatitis model of Example 1.

### Best Mode for Embodying the Invention

The present invention is explained in more detail in the following.

The therapeutic drug of the present invention comprises the aforementioned compound represented by the formula (I) as an active ingredient. Each symbol in the aforementioned formula (I) means as follows.

Halogen for R¹ or R² means fluorine, chlorine, bromine or iodine.

Lower alkyl for R¹ or R² means alkyl having 1 to 4 carbon atoms such as methyl, ethyl, propyl, isopropyl, butyl, isobutyl, tertiary butyl and the like.

Lower alkoxy for R¹ or R² means alkoxy having 1 to 4 carbon atoms such as methoxy, ethoxy, propoxy, isopropoxy, butoxy, tertiary butoxy and the like.

In the amino for R¹ or R², which is mono- or di-substituted by a group selected from lower alkyl and lower acyl, lower alkyl as the substituent means an alkyl having 1 to 4 carbon atoms such as methyl, ethyl, propyl, isopropyl, butyl, isobutyl, tertiary butyl and the like. The lower acyl as the substituent means a lower alkanoyl substituted by lower alkanoyl having 1 to 4 carbon atoms, lower alkoxycarbonyl or phenyl group and, for example, formyl, acetyl, propionyl, butyryl, methoxycarbonyl, ethoxycarbonyl, propoxycarbonyl, isopropoxycarbonyl, butoxycarbonyl, isobutoxycarbonyl, tertiary butoxycarbonyl, benzoyl, phenylacetyl and phenylpropionyl can be mentioned. The amino mono- or di-substituted by these substituents means methylamino, dimethylamino, ethylamino, diethylamino, propylamino, butylamino, acetylamino, diacetylamino, propionylamino, dipropionylamino, butyrylamino, N-methyl-N-acetylamino, N-ethyl-N-acetylamino, N-methyl-N-propionylamino, methoxycarbonylamino, ethoxycarbonylamino, propoxycarbonylamino, tertiary butoxycarbonylamino, benzoylamino, phenylacetylamino and the like.

Halogen for R³, R⁴ or R⁵ means fluorine, chlorine, bromine or iodine.

Lower alkyl for R³, R⁴ or R⁵ means alkyl having 1 to 4 carbon atoms such as methyl, ethyl, propyl, isopropyl, butyl, isobutyl, tertiary butyl and the like.

Lower alkoxy for R³, R⁴ or R⁵ means alkoxy having 1 to 4 carbon atoms such as methoxy, ethoxy, propoxy, isopropoxy, butoxy, tertiary butoxy and the like.

In the amino for R³, R⁴ or R⁵, which is mono- or di-substituted by a group selected from lower alkyl and lower acyl, lower alkyl as the substituent means alkyl having 1 to 4 carbon atoms such as methyl, ethyl, propyl, isopropyl, butyl, isobutyl, tertiary butyl and the like. Lower acyl as the substituent means a lower alkanoyl substituted by lower alkanoyl having 1 to 4 carbon atoms, lower alkoxycarbonyl or phenyl group and, for example, formyl, acetyl, propionyl, butyryl, methoxycarbonyl, ethoxycarbonyl, propoxycarbonyl, isopropoxycarbonyl, butoxycarbonyl, isobutoxycarbonyl, tertiary butoxycarbonyl, benzoyl, phenylacetyl, phenylpropionyl can be mentioned. The amino mono- or di-substituted by these substituents means methylamino, dimethylamino, ethylamino, diethylamino, propylamino, butylamino, acetylamino, diacetylamino, propionylamino, dipropionylamino, butyrylamino, N-methyl-N-acetylamino, N-ethyl-N-acetylamino, N-methyl-N-propionylamino, methoxycarbonylamino, ethoxycarbonylamino, propoxycarbonylamino, tertiary butoxycarbonylamino, benzoylamino, phenylacetylamino and the like.

Lower alkyl for R⁶ or R⁷ means alkyl having 1 to 4 carbon atoms such as methyl, ethyl, propyl, isopropyl, butyl, isobutyl, tertiary butyl and the like.

Lower alkyl substituted by 1 to 3 halogens for R⁶ or R⁷ means lower alkyl having 1 to 4 carbon atoms, which is substituted by halogen (fluorine, chlorine, bromine and the like), and fluoromethyl, trifluoromethyl, chloromethyl-, bromomethyl, 2-fluoroethyl, 2,2,2-trifluoroethyl, 2-chloroethyl, 2-bromoethyl, 3-fluoropropyl, 3-chloropropyl, 4-fluorobutyl, 4-chlorobutyl and the like can be mentioned.

Aralkyl for R⁶ or R⁷ means benzyl, 2-phenylethyl, 3-phenylpropyl and the like.

Acyl for R⁶ or R⁷ means lower alkanoyl having 1 to 4 carbon atoms or lower alkylsulfonyl having 1 to 4 carbon atoms, which is substituted by alkanoyl having 1 to 5 carbon atoms, lower alkoxycarbonyl having 1 to 4 carbon atoms, phenyl group or pyridyl group, and formyl, acetyl, propionyl, butyryl, valeryl, isovaleryl, trimethylacetyl, methoxycarbonyl, ethoxycarbonyl, propoxycarbonyl, isopropoxycarbonyl, butoxycarbonyl, isobutoxycarbonyl, tertiary butoxycarbonyl, benzoyl, nicotinoyl, isonicotinoyl, picolinoyl, phenylacetyl, phenylpropionyl, methanesulfonyl and the like can be mentioned.

Lower acyl substituted by 1 to 3 halogens for R⁶ or R⁷ means lower acyl having 1 to 4 carbon atoms which is substituted by halogen (fluorine, chlorine, bromine and the like) and fluoroacetyl, trifluoroacetyl, chloroacetyl, bromoacetyl, 3-chloropropionyl, 3-bromopropionyl, 4-chlorobutyryl, 4-bromobutyryl and the like can be mentioned.

Lower alkyl for R⁸ or R⁹ means alkyl having 1 to 4 carbon atoms and methyl, ethyl, propyl, isopropyl, n-butyl, isobutyl, secondary butyl, tertiary butyl and the like can be mentioned.

Lower alkyl for R¹⁰ or R¹¹ means alkyl having 1 to 4 carbon atoms and methyl, ethyl, propyl, isopropyl, butyl and the like can be mentioned.

Lower alkyl for R¹² or R¹³ means alkyl having 1 to 4 carbon atoms and methyl, ethyl, propyl, isopropyl, butyl and the like can be mentioned.

Alkylene formed by R¹² and R¹³ in combination means methylene, ethylene, trimethylene, tetramethylene, pentamethylene and the like.

Lower alkyl for R¹⁴ or R¹⁵ means alkyl having 1 to 4 carbon atoms such as methyl, ethyl, propyl, isopropyl, butyl and the like.

Halogen for R¹⁶ or R¹⁷ means fluorine, chlorine, bromine or iodine.

Lower alkyl for R¹⁶ or R¹⁷ means alkyl having 1 to 4 carbon atoms and methyl, ethyl, propyl, isopropyl, butyl, isobutyl, tertiary butyl and the like can be mentioned.

Pyrimidyl, thiazolyl, pyridyl, pyradyl or imidazolyl for ring A means 2-pyrimidyl, 4-pyrimidyl, 5-pyrimidyl, 2-thiazolyl, 4-thiazolyl, 5-thiazolyl, 2-pyridyl, 3-pyridyl, 4-pyridyl, 2-pyradyl and 2-imidazolyl and the like.

Particularly, a compound of the formula (I) wherein R¹, and R² are each a hydrogen, R³, R⁴, and R⁵ are the same or different and each is a halogen or a lower alkoxy, R⁶ and R⁷ are each an acyl, R⁸ and R⁹ are the same or different and each is a hydrogen or a lower alkyl, R¹⁰ and R¹¹ are each a hydrogen, R¹⁴ and R¹⁵ are each a hydrogen, and ring A is phenyl, pyrimidyl, thiazolyl or pyridyl, or pharmaceutically acceptable salt thereof is preferable.

As a pharmaceutically acceptable salt of the compound of the present invention (I), salts with inorganic acids such as hydrochloric acid, hydrogen bromide acid, hydrogen iodide acid, sulfuric acid, nitric acid, phosphoric acid and the like, salts with organic acids such as acetic acid, maleic acid, fumaric acid, benzoic acid, citric acid, succinic acid, tartaric acid, malic acid, mandelic acid, methanesulfonic acid, benzenesulfonic acid, p-toluenesulfonic acid, 10-camphorsulfonic acid and the like, and the like can be mentioned. In addition, the compound of the present invention can be converted to quaternary ammonium salt. The compound of the present invention (I) or a pharmaceutically acceptable salt thereof may be a hydrate (1 hydrate, 1/2 hydrate, 1/4 hydrate, 1/5 hydrate, 2 hydrate, 3/2 hydrate, 3/4 hydrate and the like) or a solvate. In addition, when compound (I) of the present invention has asymmetric atom(s), at least two kinds of optical isomers are present. The present invention encompasses these optical isomers and racemates thereof.

As preferable concrete compound, the compounds described in (3) and (9) within the gist of the above-mentioned present invention and pharmaceutically acceptable salts thereof can be mentioned.

Particularly, N-(1-(4-((4-(pyrimidin-2-yl)piperazin-1-yl)methyl)phenyl)cyclopropyl)acetamide and pharmaceutically acceptable salt thereof can be mentioned as preferable ones, wherein N-(1-(4-((4-(pyrimidin-2-yl)piperazin-1-yl)methyl)phenyl)cyclopropyl)acetamide hydrochloride is particularly preferable.

The above-mentioned compound can be produced according to the method described in patent reference 1 and, as regards the compound of the above-mentioned preferable compound, a specific Synthetic Example can be referred to.

According to the present invention, a therapeutic drug for non-viral hepatitis, which comprises the above-mentioned compound as an active ingredient, can be provided. As non-viral hepatitis, for example, acute hepatitis, hepatic encephalopathy, cirrhosis, primary biliary hepatitis, autoimmune hepatitis, alcoholic hepatitis, nonalchoholic steatohepatitis and the like can be mentioned. According to the present invention, moreover, a hepatic function-improving drug or a hepatoprotector for liver transplant patients can be provided.

When the above-mentioned compound is used as a therapeutic drug for non-viral hepatitis and the like, it is prepared as a general pharmaceutical agent. For example, the above-mentioned compound can be formulated in the form suitable for oral or parenteral administration such as a pharmaceutical composition obtained by mixing with a pharmaceutically acceptable carrier (excipient, binder, disintegrant, corrigent, flavor, emulsifier, diluent, solubilizer and the like), or tablet, pill, powder, granule, capsule, troche, syrup, liquid, emulsion, suspension, injection (liquid, suspension etc.), suppository, inhalant, percutaneous absorber, eye drop, nose drop, eye ointment and the like.

For production of a solid preparation, additive such as sucrose, lactose, cellulose, D-mannitol, maltitol, dextran, starches, agar, alginates, chitins, chitosans, pectins, trungums, gum arabics, gelatins, collagens, casein, albumin, calcium phosphate, sorbitol, glycine, carboxymethylcellulose, polyvinylpyrrolidone, hydroxypropylcellulose, hydroxypropylmethylcellulose, glycerol, polyethylene glycol, sodium hydrogencarbonate, magnesium stearate, talc and the like are used. Moreover, where necessary, tablet can be formulated into general coated-tablet, for example sugar-coated tablet, enteric-coated tablet, film-coated tablet, or a two-layer tablet or a multi-layer tablet.

For production of a semi-solid preparation, animal and plant oils (olive oil, corn oil, castor oil and the like), mineral oil (petrolatum, white petrolatum, solid paraffin and the like), waxes (jojoba wax, carnauba wax, bee wax and the like), partial synthesis or net synthesis glycerine fatty acid ester (lauryl acid, miristic acid, palmitic acid and the like) and the like are used. Examples of commercially available products thereof include Witepzol (manufactured by Dynamit Nobel Corporation), Pharmasol (manufactured by NOF Corporation) and the like.

For production of a liquid preparation, additives such as sodium chloride, glucose, sorbitol, glycerol, olive oil, propylene glycol, ethyl alcohol and the like can be used. Particularly, for production of an injection, sterile aqueous solutions such as saline, isotonic solution, oily liquid (e.g., sesame oil, soy bean oil) and the like are used. Where necessary, moreover, suitable suspending agent such as sodium carboxymethylcellulose, non-ionic surfactant, solubilizer (e.g., benzyl benzoate, benzyl alcohol) and the like may be used in combination.

For production of eye drops or nose drops, moreover, aqueous liquid or aqueous solution is used and sterile aqueous solution for injection can be particularly mentioned. The liquid for eye drops or nose drops may contain various additives as appropriate, such as buffer (borate buffer, acetate buffer, carbonate buffer and the like for relieving irritation are preferable), isotonicity agent, solubilizer, preservative, thickener, chelating agent, pH adjusting agent (generally, pH is preferably adjusted to about 6-8.5), fragrance and the like.

The amount of the active ingredient of these preparations is 0.1-100 wt%, suitably 1-50 wt%, of the preparation. While the dose may vary depending on the condition, body weight, age and the like of patients, in the case of oral administration, it is generally 0.01-50 mg/day for an adult, which is preferably administered at once or in several portions.

### Example

The present invention is explained in more detail in the following by referring to Example, which is not to be construed as limitative as long as it does not deviate from the subject matter of the present invention.

The test compound used below was N-(1-(4-((4-(pyrimidin-2-yl)piperazin-1-yl)methyl)phenyl)cyclopropyl)acetamide hydrochloride produced according to the method described in patent reference 1, Example 71.

### Experimental Example 1: Action on concanavalin A-induced hepatitis

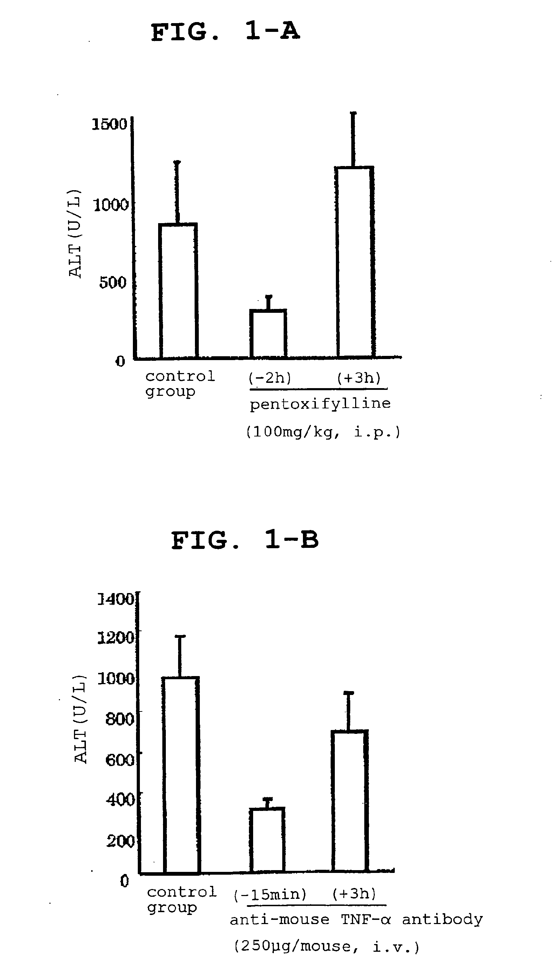

20 mg/kg of concanavalin A (Con A, type V, manufactured by Sigma) was intravenously administered to female BALB/c mouse (purchased from Charles River Laboratories JAPAN, INC.). Since plasma alanyltransferase (ALT) shows a peak 8 hr after Con A administration, plasma ALT was measured at this time point using FUJI Drichem 3500. The administration time in the prophylactic administration test of pentoxifylline (manufactured by Sigma) or anti-mouse TNF-α antibody (manufactured by Genzyme-Techne) was set according to the method of Shirin et al. [Journal of Hepatology, 29, 60-67 (1998)] or Gantner et al. [Hepatology, 21, 190-198 (1995)]. The administration time in the therapeutic administration test was always set to 3 hr after Con A injection. To be specific, pentoxifylline (100 mg/kg) was intraperitoneally administered 2 hr before or 3 hr after Con A injection, anti-mouse TNF-α antibody (250 µg) was intravenously administrated 15 minutes before or 3 hr after Con A injection, and plasma ALT was similarly measured 8 hr later.
The results are shown in Fig. 1.

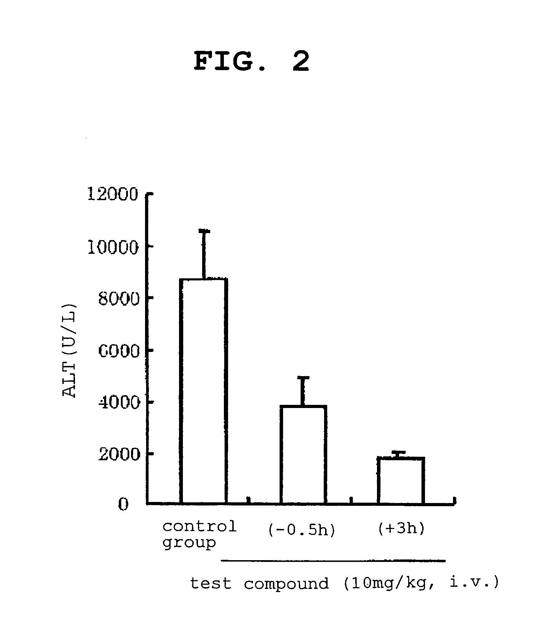

In addition, the test compound (10 mg/kg) was intravenously administrated 15 minutes before or 3 hr after Con A injection, and plasma ALT concentration was similarly measured.
The results are shown in Fig. 2.
The results are shown in mean ± standard error.

From these results, it is clear that the test compound shows a therapeutic effect in the hepatitis model, unlike pentoxifylline and anti-TNF-α antibody.

### Industrial Applicability

According to the present invention, a novel therapeutic drug for non-viral hepatitis can be provided.
While the present invention has been described in detail by referring to a particular embodiment, it will, however, be evident for those of ordinary skill in the art that various modifications and changes may be made without departing from the intention and scope of the present invention.
This application is based on application No. 2004-131116 filed in Japan on April 27, 2004, the contents of which are incorporated hereinto by reference.

## Claims

1. A therapeutic drug for non-viral hepatitis comprising, as an active ingredient, a piperazine compound represented by the following formula (I) 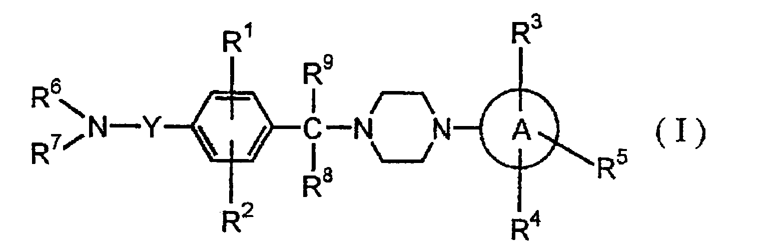 wherein R¹ and R² are the same or different and each is an amino, a nitro, a hydroxy or a cyano, which is mono- or di-substituted by a group selected from a hydrogen, a halogen, a lower alkyl, a lower alkoxy, an amino, a lower alkyl and a lower acyl;
R³, R⁴ and R⁵ are the same or different and each is an amino or a hydroxy, which is mono- or di-substituted by a group selected from a hydrogen, a halogen, a lower alkyl, a lower alkoxy, a nitro, an amino, a lower alkyl and a lower acyl;
R⁶ and R⁷ are the same or different and each is a hydrogen, a lower alkyl, a lower alkyl substituted by 1 to 3 halogens, an aralkyl, an acyl or a lower acyl substituted by 1 to 3 halogens;
R⁸ and R⁹ are the same or different and each is a hydrogen or a lower alkyl; and
Y is a group represented by 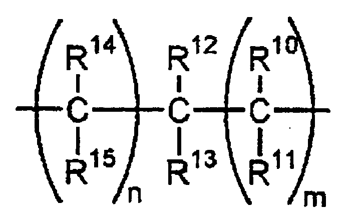 wherein R¹⁰ and R¹¹ are the same or different and each is a hydrogen or a lower alkyl;
R¹² and R¹³ are the same or different and each is a hydrogen or a lower alkyl, or R¹² and R¹³ in combination form alkylene;
R¹⁴ and R¹⁵ are the same or different and each is a hydrogen or a lower alkyl;
m is an integer of 0-2, n is an integer of 0-2, and 0≤m+n≤2; and
ring A is a phenyl, a pyrimidyl, a thiazolyl, a pyridyl, a pyradyl or an imidazolyl, or a pharmaceutically acceptable salt thereof.

2. The therapeutic drug of claim 1, wherein the active ingredient is a compound of the formula (I) wherein R¹ and R² are each a hydrogen, R³, R⁴ and R⁵ are the same or different and each is a halogen or a lower alkoxy, R⁶ and R⁷ are each an acyl, R⁸ and R⁹ are the same or different and each is a hydrogen or a lower alkyl, R¹⁰ and R¹¹ are each a hydrogen, R¹⁴ and R¹⁵ are each a hydrogen, ring A is a phenyl, a pyrimidyl, a thiazolyl or a pyridyl, or a pharmaceutically acceptable salt thereof.

3. The therapeutic drug of claim 1, wherein the active ingredient is the following compound:
N-(4-((4-phenylpiperazin-1-yl)methyl)phenylmethyl)acetamide;
N-(4-((4-(4-fluorophenyl)piperazin-1-yl)methyl)phenylmethyl)acetamide;
N-(4-((4-(2-fluorophenyl)piperazin-1-yl)methyl)phenylmethyl)acetamide;
N-(3-(4-((4-phenylpiperazin-1-yl)methyl)phenyl)propyl)acetamide;
N-(4-((4-(2,4-difluorophenyl)piperazin-1-yl)methyl)phenylmethyl)acetamide;
N-(4-(1-(4-(4-fluorophenyl)piperazin-1-yl)ethyl)phenylmethyl)acetamide;
N-(4-((4-(pyridin-2-yl)piperazin-1-yl)methyl)phenylmethyl)acetamide;
N-(4-((4-(pyrimidin-2-yl)piperazin-1-yl)methyl)phenylmethyl)acetamide;
N-(4-((4-(pyridin-3-yl)piperazin-1-yl)methyl)phenylmethyl)acetamide;
N-(4-((4-(thiazol-2-yl)piperazin-1-yl)methyl)phenylmethyl)acetamide;
N-(1-methyl-1-(4-((4-(4-fluorophenyl)piperazin-1-yl)methyl)phenyl)ethyl)acetamide;
N-(1-(4-((4-(2,4-difluorophenyl)piperazin-1-yl)methyl)phenyl)ethyl)acetamide;
N-(1-(4-((4-(2,4-difluorophenyl)piperazin-1-yl)methyl)phenyl)propyl)acetamide;
N-(1-(4-((4-(4-fluorophenyl)piperazin-1-yl)methyl)phenyl)ethyl)acetamide;
N-(1-(4-((4-phenylpiperazin-1-yl)methyl)phenyl)cyclopropyl)acetamide;
N-(1-(4-((4-(4-fluorophenyl)piperazin-1-yl)methyl)phenyl)cyclopropyl)acetamide;
N-(1-(4-((4-(2,4-difluorophenyl)piperazin-1-yl)methyl)phenyl)cyclopropyl)acetamide;
N-(4-((4-(6-fluoropyridin-2-yl)piperazin-1-yl)methyl)phenylmethyl)acetamide;
N-(4-((4-(pyrimidin-2-yl)piperazin-1-yl)methyl)phenylmethyl)acetamide;
N-(1-(4-(1-(4-phenylpiperazin-1-yl)ethyl)phenyl)-1-methylethyl)acetamide;
N-(1-(4-(1-(4-(pyrimidin-2-yl)piperazin-1-yl)ethyl)phenyl)cyclopropyl)acetamide;
N-(1-(4-((4-(pyrimidin-2-yl)piperazin-1-yl)methyl)phenyl)cyclopropyl)acetamide;
N-(1-(4-((4-(pyrimidin-2-yl)piperazin-1-yl)methyl)phenyl)ethyl)acetamide;
N-(4-((4-(4,6-dimethoxypyrimidin-2-yl)piperazin-1-yl)methyl)phenylmethyl)acetamide;
N-(1-methyl-1-(4-((4-(pyrimidin-2-yl)piperazin-1-yl)methyl)phenyl)ethyl)acetamide;
N-(1-methyl-1-(4-((4-(thiazol-2-yl)piperazin-1-yl)methyl)phenyl)ethyl)acetamide;
N-(1-(4-((4-(thiazol-2-yl)piperazin-1-yl)methyl)phenyl)cyclopropyl)acetamide;
N-(1-(4-((4-(thiazol-2-yl)piperazin-1-yl)methyl)phenyl)ethyl)acetamide;
N-(1-(4-((4-(6-fluoropyridin-2-yl)piperazin-1-yl)methyl)phenyl)-1-methylethyl)acetamide;
N-(1-(4-((4-(6-fluoropyridin-2-yl)piperazin-1-yl)methyl)phenyl)ethyl)acetamide;
N-(1-(4-((4-(pyridin-2-yl)piperazin-1-yl)methyl)phenyl)ethyl)acetamide;
N-(1-methyl-1-(4-((4-(pyridin-2-yl)piperazin-1-yl)methyl)phenyl)ethyl)acetamide;
N-(1-(4-(1-(4-(pyridin-2-yl)piperazin-1-yl)ethyl)phewyl)-1-methylethyl)acetamide;
N-(1-(4-((4-(pyridin-2-yl)piperazin-1-yl)methyl)phenyl)cyclopropyl)acetamide;
N-(1-(4-((4-(6-fluoropyridin-2-yl)piperazin-1-yl)methyl)phenyl)cyclopropyl)acetamide;
(S)-N-(1-(4-((4-(4-fluorophenyl)piperazin-1-yl)methyl)phenyl)ethyl)acetamide;
(R)-N-(1-(4-((4-(4-fluorophenyl)piperazin-1-yl)methyl)phenyl)ethyl)acetamide;
N-(4-(1-(4-(pyridin-2-yl)piperazin-1-yl)ethyl)phenylmethyl)acetamide;
N-(1-(4-(1-(4-(6-fluoropyridin-2-yl)piperazin-1-yl)ethyl)phenyl)-1-methylethyl)acetamide; or
N-(1-(4-(1-(4-(6-fluoropyridin-2-yl)piperazin-1-yl)ethyl)phenyl)methyl)acetamide,
or a pharmaceutically acceptable salt thereof.

4. The therapeutic drug of claim 1, wherein the active ingredient is N-(1-(4-((4-(pyrimidin-2-yl)piperazin-1-yl)methyl)phenyl)cyclopropyl)acetamide, or a pharmaceutically acceptable salt thereof.

5. The therapeutic drug of claim 1, wherein the active ingredient is N-(1-(4-((4-(pyrimidin-2-yl)piperazin-1-yl)methyl)phenyl)cyclopropyl)acetamide hydrochloride.

6. The therapeutic drug of any of claims 1 to 5, wherein the non-viral hepatitis is selected from acute hepatitis, hepatic encephalopathy, cirrhosis, primary biliary hepatitis, autoimmune hepatitis, alcoholic hepatitis and nonalchoholic steatohepatitis.

7. A hepatic function-improving drug or hepatoprotector for liver transplant patient, which comprises, as an active ingredient, a piperazine compound represented by the following formula (I) 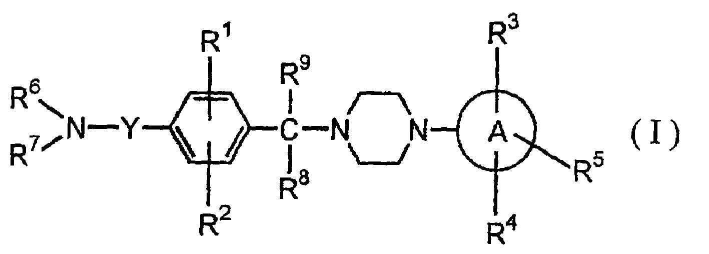 wherein R¹ and R² are the same or different and each is an amino, a nitro, a hydroxy or a cyano, which is mono- or di-substituted by a group selected from a hydrogen, a halogen, a lower alkyl, a lower alkoxy, an amino, a lower alkyl and a lower acyl;
R³, R⁴ and R⁵ are the same or different and each is an amino or hydroxy, which is mono- or di-substituted by a group selected from a hydrogen, a halogen, a lower alkyl, a lower alkoxy, a nitro, an amino, a lower alkyl and lower an acyl;
R⁶ and R⁷ are the same or different and each is a hydrogen, a lower alkyl, a lower alkyl substituted by 1 to 3 halogens, an aralkyl, an acyl or a lower acyl substituted by 1 to 3 halogens;
R⁸ and R⁹ are the same or different and each is a hydrogen or a lower alkyl;
Y is 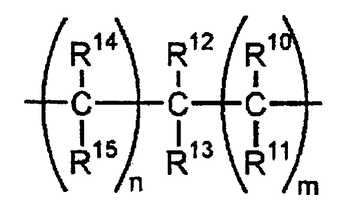 wherein R¹⁰ and R¹¹ are the same or different and each is a hydrogen or a lower alkyl;
R¹² and R¹³ are the same or different and each is a hydrogen or a lower alkyl, or R¹² and R¹³ in combination form alkylene;
R¹⁴ and R¹⁵ are the same or different and each is a hydrogen or a lower alkyl;
m is an integer of 0-2, n is an integer of 0-2, and 0≤m+n≤2; and
ring A is a phenyl, a pyrimidyl, a thiazolyl, a pyridyl, a pyradyl or an imidazolyl,
or a pharmaceutically acceptable salt thereof.

8. The hepatic function-improving drug or hepatoprotector of claim 7, wherein the active ingredient is a compound of the formula (I), wherein R¹ and R² are each a hydrogen, R³, R⁴ and R⁵ are the same or different and each is a halogen or a lower alkoxy, R⁶ and R⁷ are each an acyl, R⁸ and R⁹ are the same or different and each is a hydrogen or a lower alkyl, R¹⁰ and R¹¹ are each a hydrogen, R¹⁴ and R¹⁵ are each a hydrogen, ring A is a phenyl, a pyrimidyl, a thiazolyl or a pyridyl, or a pharmaceutically acceptable salt thereof.

9. The hepatic function-improving drug or hepatoprotector of claim 7, wherein the active ingredient is the following compound:
N-(4-((4-phenylpiperazin-1-yl)methyl)phenylmethyl)acetamide;
N-(4-((4-(4-fluorophenyl)piperazin-1-yl)methyl)phenylmethyl)acetamide;
N-(4-((4-(2-fluorophenyl)piperazin-1-yl)methyl)phenylmethyl)acetamide;
N-(3-(4-((4-phenylpiperazin-1-yl)methyl)phenyl)propyl)acetamide;
N-(4-((4-(2,4-difluorophenyl)piperazin-1-yl)methyl)phenylmethyl)acetamide;
N-(4-(1-(4-(4-fluorophenyl)piperazin-1-yl)ethyl)phenylmethyl)acetamide;
N-(4-((4-(pyridin-2-yl)piperazin-1-yl)methyl)phenylmethyl)acetamide;
N-(4-((4-(pyrimidin-2-yl)piperazin-1-yl)methyl)phenylmethyl)acetamide;
N-(4-((4-(pyridin-3-yl)piperazin-1-yl)methyl)phenylmethyl)acetamide;
N-(4-((4-(thiazol-2-yl)piperazin-1-yl)methyl)phenylmethyl)acetamide;
N-(1-methyl-1-(4-((4-(4-fluorophenyl)piperazin-1-yl)methyl)phenyl)ethyl)acetamide;
N-(1-(4-((4-(2,4-difluorophenyl)piperazin-1-yl)methyl)phenyl)ethyl)acetamide;
N-(1-(4-((4-(2,4-difluorophenyl-)piperazin-1-yl)methyl)phenyl)propyl)acetamide;
N-(1-(4-((4-(4-fluorophenyl)piperazin-1-yl)methyl)phenyl)ethyl)acetamide;
N-(1-(4-((4-phenylpiperazin-1-yl)methyl)phenyl)cyclopropyl)acetamide;
N-(1-(4-((4-(4-fluorophenyl)piperazin-1-yl)methyl)phenyl)cyclopropyl)acetamide;
N-(1-(4-((4-(2,4-difluorophenyl)piperazin-1-yl)methyl)phenyl)cyclopropyl)acetamide;
N-(4-((4-(6-fluoropyridin-2-yl)piperazin-1-yl)methyl)phenylmethyl)acetamide;
N-(4-((4-(pyrimidin-2-yl)piperazin-1-yl)methyl)phenylmethyl)acetamide;
N-(1-(4-(1-(4-phenylpiperazin-1-yl)ethyl)phenyl)-1-methylethyl)acetamide;
N-(1-(4-(1-(4-(pyrimidin-2-yl)piperazin-1-yl)ethyl)phenyl)cyclopropyl)acetamide;
N-(1-(4-((4-(pyrimidin-2-yl)piperazin-1-yl)methyl)phenyl)cyclopropyl)acetamide;
N-(1-(4-((4-(pyrimidin-2-yl)piperazin-1-yl)methyl)phenyl)ethyl)acetamide;
N-(4-((4-(4,6-dimethoxypyrimidin-2-yl)piperazin-1-yl)methyl)phenylmethyl)acetamide;
N-(1-methyl-1-(4-((4-(pyrimidin-2-yl)piperazin-1-yl)methyl)phenyl)ethyl)acetamide;
N-(1-methyl-1-(4-((4-(thiazol-2-yl)piperazin-1-yl)methyl)phenyl)ethyl)acetamide;
N-(1-(4-((4-(thiazol-2-yl)piperazin-1-yl)methyl)phenyl)cyclopropyl)acetamide;
N-(1-(4-((4-(thiazol-2-yl)piperazin-1-yl)methyl)phenyl)ethyl)acetamide;
N-(1-(4-((4-(6-fluoropyridin-2-yl)piperazin-1-yl)methyl)phenyl)-1-methylethyl)acetamide;
N-(1-(4-((4-(6-fluoropyridin-2-yl)piperazin-1-yl)methyl)-phenyl)ethyl)acetamide;
N-(1-(4-((4-(pyridin-2-yl)piperazin-1-yl)methyl)phenyl)ethyl)acetamide;
N-(1-methyl-1-(4-((4-(pyridin-2-yl)piperazin-1-yl)methyl)phenyl)ethyl)acetamide;
N-(1-(4-(1-(4-(pyridin-2-yl)piperazin-1-yl)ethyl)phenyl)-1-methylethyl)acetamide;
N-(1-(4-((4-(pyridin-2-yl)piperazin-1-yl)methyl)phenyl)cyclopropyl)acetamide;
N-(1-(4-((4-(6-fluoropyridin-2-yl)piperazin-1-yl)methyl)phenyl)cyclopropyl)acetamide;
(S)-N-(1-(4-((4-(4-fluorophenyl)piperazin-1-yl)methyl)phenyl)ethyl)acetamide;
(R)-N-(1-(4-((4-(4-fluorophenyl)piperazin-1-yl)methyl)phenyl)ethyl)acetamide;
N-(4-(1-(4-(pyridin-2-yl)piperazin-1-yl)ethyl)phenylmethyl)acetamide;
N-(1-(4-(1-(4-(6-fluoropyridin-2-yl)piperazin-1-yl)ethyl)phenyl)-1-methylethyl)acetamide; or
N-(1-(4-(1-(4-(6-fluoropyridin-2-yl)piperazin-1-yl)ethyl)phenyl)methyl)acetamide,
or a pharmaceutically acceptable salt thereof.

10. The hepatic function-improving drug or hepatoprotector of claim 7, wherein the active ingredient is N-(1-(4-((4-(pyrimidin-2-yl)piperazin-1-yl)methyl)phenyl)cyclopropyl)acetamide or a pharmaceutically acceptable salt thereof.

11. The hepatic function-improving drug or hepatoprotector of claim 7, wherein the active ingredient is N-(1-(4-((4-(pyrimidin-2-yl)piperazin-1-yl)methyl)phenyl)cyclopropyl)acetamide hydrochloride.
